(19) Europäisches Patentamt
European Patent Office
Office européen des brevets

(11) **EP 4 303 176 B1**

(12) **EUROPEAN PATENT SPECIFICATION**

(45) Date of publication and mention
of the grant of the patent:
**19.11.2025 Bulletin 2025/47**

(21) Application number: **22792066.7**

(22) Date of filing: **22.04.2022**

(51) International Patent Classification (IPC):
**A61K 9/51** (2006.01)   **C12N 15/88** (2006.01)
**B82B 3/00** (2006.01)   **B82Y 40/00** (2011.01)
**B01F 23/41** (2022.01)   **B01F 33/30** (2022.01)
**B01J 19/00** (2006.01)

(52) Cooperative Patent Classification (CPC):
**C12N 15/88; A61K 9/5123; A61K 9/5192;**
**A61K 31/7105; A61K 31/713; B01F 23/41;**
**B01F 33/30; B01J 19/0093;** A61K 39/39;
A61K 2039/53; A61K 2039/55555;
B01J 2219/00889; B82Y 30/00; B82Y 40/00

(86) International application number:
**PCT/KR2022/005796**

(87) International publication number:
**WO 2022/225368 (27.10.2022 Gazette 2022/43)**

(54) **LIPID NANOPARTICLE PREPARATION METHOD AND PREPARATION APPARATUS THEREFOR**

VERFAHREN ZUR HERSTELLUNG VON LIPIDNANOPARTIKELN UND VORRICHTUNG ZUR HERSTELLUNG DAVON

MÉTHODE DE PRÉPARATION DE NANOPARTICULES LIPIDIQUES ET APPAREIL DE PRÉPARATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2021 KR 20210052233**

(43) Date of publication of application:
**10.01.2024 Bulletin 2024/02**

(73) Proprietor: **Inventage Lab Inc.**
**Gyeonggi-do 13403 (KR)**

(72) Inventors:
 • **PARK, Hyochan**
  **Seongnam-si, Gyeonggi-do 13438 (KR)**
 • **CHON, Chan Hee**
  **Seongnam-si, Gyeonggi-do 13165 (KR)**
 • **KIM, Donghoon**
  **Seongnam-si, Gyeonggi-do 13530 (KR)**
 • **KIM, Ju Hee**
  **Seongnam-si, Gyeonggi-do 13494 (KR)**

(74) Representative: **BCKIP Part mbB**
**MK1**
**Landsbergerstraße 98, 3.Stock**
**80339 München (DE)**

(56) References cited:
 WO-A1-2018/190423   WO-A1-2022/225368
 JP-A- 2016 028 031   KR-A- 20140 097 276
 KR-A- 20150 127 580   KR-A- 20170 126 944

 • **BUSCHMANN MICHAEL D., CARRASCO MANUEL J., ALISHETTY SUMAN, PAIGE MIKELL, ALAMEH MOHAMAD GABRIEL, WEISSMAN DREW: "Nanomaterial Delivery Systems for mRNA Vaccines", VACCINES, vol. 9, no. 1, 65, 1 January 2021 (2021-01-01), M D P I AG, CH, XP055927791, ISSN: 2076-393X, DOI: 10.3390/ vaccines9010065**

- KIMURA NIKO, MAEKI MASATOSHI, SATO YUSUKE, ISHIDA AKIHIKO, TANI HIROFUMI, HARASHIMA HIDEYOSHI, TOKESHI MANABU: "Development of a Microfluidic-Based Post-Treatment Process for Size-Controlled Lipid Nanoparticles and Application to siRNA Delivery", APPLIED MATERIALS & INTERFACES, vol. 12, no. 30, 29 July 2020 (2020-07-29), American Chemical Society, US, pages 34011 - 34020, XP055978520, ISSN: 1944-8244, DOI: 10.1021/acsami.0c05489

## Description

## Technical Field

The present disclosure relates to a method for manufacturing lipid nanoparticles (LNP) and an apparatus for manufacturing the same.

## Background Art

Lipid nanoparticles (LNP) are an effective drug delivery system for cell-impermeable therapeutic nucleic acids, proteins, and biologically active compounds such as peptides.

Vaccines are generally subdivided into "first generation", "second generation" and "third generation" vaccines, and gene vaccines, that is, vaccines for genetic vaccination are generally understood as a "third generation" vaccine. Gene vaccines are typically formed with genetically modified nucleic acid molecules allowing an expression of peptide or protein (antigen) fragments specific for pathogens or tumor antigens in vivo. When a gene vaccine is administered to a patient, it is expressed after uptake by target cells. Expression of the administered nucleic acid brings production of an encoded protein. When such a protein is recognized as a foreign substance by a patient's immune system, an immune response is triggered.

In the context of genetic vaccination, RNA as well as DNA may be used as nucleic acid molecules for administration. DNA is known to be relatively stable and easy to handle.

However, the use of DNA carries a risk of unwanted insertion of administered DNA fragments potentially causing mutagenic events such as loss of function of damaged genes into patient's genome.

By using RNA instead of DNA for genetic vaccination, risks of unwanted genome integration and anti-DNA antibody generation are minimized or prevented. However, RNA may be readily degraded by ubiquitous RNases and is highly unstable, thereby having problems of impermeability, fragility and immunogenicity.

Although much progress has been made over the past few years, lipid nanoparticle formulations have been used as an efficient method for mRNA vaccination capable of inducing an adaptive immune response.

The lipid nanoparticle formulations may improve in vivo nucleic acid delivery.

A drug delivery system using the lipid nanoparticles is a multi-component formulation including an ionizable lipid, a non-ionizable lipid, a neutral lipid and a conjugated lipid. A cationic ionizable lipid binds to anionic nucleic acid, whereas other components support stable self-assembly of the lipid nanoparticles.

The lipid nanoparticles are manufactured in an optimal drug:lipid ratio, and protect nucleic acid from degradation and elimination in serum, are suitable for systemic or local delivery, and are capable of providing intracellular delivery of nucleic acid.

When the lipid nanoparticles are manufactured using a traditional manufacturing method, sizes of manufactured particles are not uniform, which causes a problem of reducing production efficiency such as requiring a separate sorting process.

Development of a process for manufacturing lipid nanoparticles having a uniform diameter has been required in order to address such an issue and increase production efficiency.

[Prior Art Documents]

[Patent Documents]

(Patent Document) KR 10-2019-0093816 A1;

JP 2016-028031 A;
KR 10-2017-0126944 A ;
WO 2018-190423 A1.

[Non-Patent Documents]

MD Buschmann et al.: "Nanomaterial Delivery Systems for mRNA Vaccines" in Vaccines 2021, 9, 65, 1 to 30;
N Kimura et al.: "Development of a Microfluidic-Based Post-Treatment Process for Size-Controlled Lipid Nanoparticles and Application to siRNA Delivery" in ACS Appl. Mater. Interfaces, 2020, 12, 30, 34011 to 34020.

## DISCLOSURE

### Technical Problem

The present disclosure is directed to providing a method for manufacturing lipid nanoparticles and an apparatus for manufacturing the same.

The present disclosure is also directed to providing an apparatus for manufacturing lipid nanoparticles, the apparatus not requiring a separate process for sorting manufactured lipid nanoparticles, thereby increasing a production yield in a subsequent eradication filtration process and the like.

The present disclosure is also directed to providing a method for manufacturing uniform-sized lipid nanoparticles, the method capable of lowering a content of ionizable lipid that causes a problem of toxicity when injected into the body by deviating from an existing optimal drug and lipid ratio.

### Technical Solution

In view of the above, one embodiment of the present disclosure provides a method for manufacturing lipid nanoparticles, the method including: preparing an aqueous phase solution including nucleic acid; preparing a first oil phase solution by dissolving an ionizable lipid in an organic solution; preparing a second oil phase solution by dissolving a non-ionizable lipid, a neutral lipid and a conjugated lipid in an organic solution; injecting and flowing the aqueous phase solution into a first channel; injecting and flowing the first oil phase solution into a second channel; the aqueous phase solution and the first oil phase solution intersecting with each other to flow through a stirring channel as a first mixture solution; flowing the second oil phase solution to a third channel connected to the stirring channel to intersect with the first mixture solution and be mixed as a second mixture solution; and forming lipid nanoparticles including nucleic acid by passing the second mixture solution through a stirring unit in the stirring channel.

The stirring channel may include a mixing module of the stirring unit and a non-stirring unit.

The mixing module is formed in plural in the stirring channel, and an $n^{th}$ mixing module is formed in order based on a fluid flow direction of the stirring channel, and the $n^{th}$ mixing module may mean an order of the mixing module repeatedly formed in the stirring channel.

The third channel forms an intersection between the first mixing module to the fifth mixing module to be coupled with the stirring channel, and the first mixture solution and the second oil phase solution may intersect to form a laminar flow.

The lipid nanoparticles may have a uniform spherical shape, and may have a polydispersity index of 0.2 or less.

The ionizable lipid may be included in an amount of 10 mol% to 30 mol% with respect to the total lipid weight in the lipid nanoparticles.

The ionizable lipid and the nucleic acid may have a weight ratio of 3:1 to 50:1.

The nucleic acid may be selected from the group consisting of RNA, DNA, siRNA (short interfering RNA), mRNA (messenger RNA) aptamer, antisense ODN (antisense oligodeoxynucleotide), antisense RNA, ribozyme, DNAzyme and mixtures thereof.

The non-ionizable lipid may be selected from the group consisting of DSPC (distearoylphosphatidylcholine), DOPE (dioleolphosphatidyl ethanolamine), DPPE (bis(diphenylphosphino)ethane), diacyl phosphatidylcholine, diacylphosphatidylethanolamine, diacylphosphatidylserine and mixtures thereof.

The neutral lipid may be selected from the group consisting of polyethylene glycol 2000 distearoylphosphatidylethanolamine (PEG(2000) DSPE), DMG-PEG, PEG-DMPE, DPPE-PEG, DPG-PEG, PEG-DOPE and mixtures thereof.

The conjugated lipid may be selected from the group consisting of phospholipid, cholesterol, tocopherol and mixtures thereof.

Lipid nanoparticles including a low concentration of ionizable lipid according to another embodiment of the present disclosure may be manufactured using the above-described manufacturing method.

An apparatus for manufacturing lipid nanoparticles including a low concentration of ionizable lipid according to another embodiment of the present disclosure includes: a first channel through which an aqueous phase solution including nucleic acid flows; a second channel through which a first oil phase solution including an ionizable lipid flows; a third channel through which a second oil phase solution including a non-ionizable lipid, a neutral lipid and a conjugated lipid flows; and a stirring channel, wherein the first channel and the second channel form a first intersection, the intersection is connected to the stirring channel, and the stirring channel may include a mixing module of a stirring unit and a non-stirring unit.

The mixing module may be formed in plural in the stirring channel, and a $n^{th}$ mixing module may be formed in order based on a fluid flow direction of the stirring channel, and the $n^{th}$ mixing module may mean an order of the mixing module repeatedly formed in the stirring channel.

The number of the mixing modules included in the stirring channel may be from 3 to 70.

The third channel may form an intersection between the first mixing module to the fifth mixing module to be coupled with the stirring channel.

The mixing module may have a length of 1 mm to 5 mm based on a fluid flow direction in the stirring channel.

The stirring unit and the non-stirring unit may have a length ratio of 45:1 to 5:0.3 based on a fluid flow direction in the stirring channel.

A groove may be formed in the stirring unit in order to mix the introduced fluids.

The groove of the stirring unit may generate chaotic mixing in order to increase mixing efficiency of a laminar flow in the stirring channel.

The groove may have a shape of rectangle, semicircle or triangle.

## Advantageous Effects

The present disclosure does not require a separate process for sorting manufactured lipid nanoparticles, thereby capable of increasing a production yield in a subsequent eradication filtration process and the like.

In addition, by deviating from an existing optimal drug and lipid ratio, a content of ionizable lipid that causes a problem of toxicity when injected into the body can be lowered, and uniform-sized lipid nanoparticles can be manufactured.

## Brief Description of Drawings

FIG. 1 is a diagram of a manufacturing apparatus according to one embodiment of the present disclosure.

FIG. 2 is a diagram of an existing apparatus for manufacturing lipid nanoparticles according to one embodiment of the present disclosure.

FIG. 3 is a photograph of a manufacturing apparatus according to one embodiment of the present disclosure.

FIG. 4 shows a result of measuring PDI of lipid nanoparticles manufactured by one embodiment of the present disclosure.

FIG. 5 is a Cryo-EM image of a lipid nanoparticle according to one embodiment of the present disclosure.

FIG. 6 shows a result of measuring a mixing index rate (%) according to a flow rate ratio between an aqueous phase solution and a first oil phase solution according to one embodiment of the present disclosure.

## Best Mode

The present disclosure relates to a method for manufacturing lipid nanoparticles, the method including: preparing an aqueous phase solution including nucleic acid; preparing a first oil phase solution by dissolving an ionizable lipid in an organic solution; preparing a second oil phase solution by dissolving a non-ionizable lipid, a neutral lipid and a conjugated lipid in an organic solution; injecting and flowing the aqueous phase solution into a first channel; injecting and flowing the first oil phase solution into a second channel; the aqueous phase solution and the first oil phase solution intersecting with each other to flow through a stirring channel as a first mixture solution; flowing the second oil phase solution to a third channel connected to the stirring channel to intersect with the first mixture solution and be mixed as a second mixture solution; and forming lipid nanoparticles including nucleic acid by passing the second mixture solution through a stirring unit in the stirring channel.

Hereinafter, embodiments of the present disclosure will be described in detail so that those skilled in the art may readily implement the present disclosure. However, the present disclosure may be embodied in various different forms, and is not limited to the embodiments described herein.

mRNA, short for messenger ribonucleic acid, is an intermediate connecting DNA and protein in a process in which DNA having genetic information becomes mRNA and protein is synthesized using the same.

Due to COVID-19, attention and development are focused on mRNA vaccines. mRNA vaccines have several advantages over other types of vaccines. The biggest advantage of mRNA vaccines is that lipid nanoparticles (LNP) including mRNA correspond to a platform technology, allowing rapid technology development against viruses with frequent mutations such as COVID-19.

Specifically, mRNA may be prepared by identifying a protective protein antigen and sequencing a gene for the antigen. When new mRNA is prepared using such a method and formulation design and manufacturing process of existing mRNA vaccines are used, rapid mRNA vaccine preparation is possible. This means that, since mRNAs that encode different antigens are very similar chemically and physically, formulation design and a manufacturing process of a new mRNA vaccine may be performed in the same steps as formulation design and a manufacturing process of existing mRNA vaccines.

By the negative charge of the phosphate group, mRNA is generally a polyanionic macromolecule in the pH range used for parenteral use. Using electrical properties of negatively charged mRNA as above, lipid nanoparticles may be prepared using a positively charged ionizable lipid (ionizable lipid or cationic lipid). Specifically, an ionizable lipid is a positively charged lipid, and strongly binds to negatively charged mRNA through electrical attraction. In addition to the ionizable lipid, a non-ionizable lipid, a neutral lipid and a conjugated lipid are further included to form lipid nanoparticles.

It is disclosed that a nucleic acid-lipid particle of US 9364435 B2 includes (a) a nucleic acid, (b) a cationic lipid, (c) a non-cationic lipid and (d) a conjugated lipid, and, based on the total lipid content in the particle, the cationic lipid is included in an amount of 50 mol% to 85 mol%, the non-cationic lipid is included in an amount of 13 mol% to 49.5 mol%, and the conjugated lipid is included in an amount of 0.5 mol% to 2 mol%.

In addition, it is disclosed that a nucleic acid-lipid particle of EP 2279254 B1 includes, based on the total lipid content in the particle, a cationic lipid in an amount of 50 mol% to 65 mol%, a non-cationic lipid in an amount of 49.5 mol% or less, cholesterol or a derivative thereof in an amount of 30 mol% to 40 mol%, and a conjugated lipid in an amount of 0.5 mol% to 2 mol%.

Nano-lipid particles including mRNA as above are identified to include a large amount of ionizable lipid (cationic lipid) in the particles.

However, there still remains some concerns about therapeutic safety of lipid nanoparticles (LNP) including mRNA as above due to toxicity in vitro and in vivo. Such toxicity arises primarily on the basis of non-specific charge interactions. In other words, a toxicity problem of positively charged ionizable lipid is becoming an issue, and studies to complement this problem are ongoing.

An attempt to reduce the used amount of cationic ionizable lipid is most intuitive, however, when using a low concentration of ionizable lipid below the threshold, this causes a problem in that manufactured lipid nanoparticles (LNP) are not uniform in the size. When lipid nanoparticles (LNP) that are not uniform in the size are manufactured, a process for removing lipid nanoparticles (LNP) of unwanted sizes is further required, leading to large losses even in a subsequent eradication filtration process and the like. This causes a significant decrease in the production yield of lipid nanoparticles including mRNA.

The present disclosure relates to a method for manufacturing lipid nanoparticles including nucleic acid, and is directed to providing a manufacturing method capable of manufacturing lipid nanoparticles with a uniform particle size.

In addition, as described above, lipid nanoparticles having a uniform diameter are manufactured while lowering the content of ionizable lipid causing a problem of toxicity. Even when the content is within the lipid content range for manufacturing existing lipid nanoparticles or the ionizable lipid content range is reduced, particles with a uniform diameter may be manufactured by using a method different from an existing manufacturing method.

Specifically, the method may include: preparing an aqueous phase solution including nucleic acid; preparing a first oil phase solution by dissolving an ionizable lipid in an organic solution; preparing a second oil phase solution by dissolving a non-ionizable lipid, a neutral lipid and a conjugated lipid in an organic solution; injecting and flowing the aqueous phase solution into a first channel; injecting and flowing the first oil phase solution into a second channel; the aqueous phase solution and the first oil phase solution intersecting with each other to flow through a stirring channel as a first mixture solution; flowing the second oil phase solution to a third channel connected to the stirring channel to intersect with the first mixture solution and to mixed as a second mixture solution; and forming lipid nanoparticles including nucleic acid by passing the second mixture solution through a stirring unit in the stirring channel.

Specifically, nucleic acid is mixed with a solvent to prepare an aqueous phase solution. The solvent is a citric acid solution and has a pH of 3.0, however, the solvent is not limited to the example, and solvents capable of being mixed with nucleic acid to manufacture lipid nanoparticles may all be used without limit.

The nucleic acid may be selected from the group consisting of RNA, DNA, siRNA (short interfering RNA), mRNA (messenger RNA) aptamer, antisense ODN (antisense oligodeoxynucleotide), antisense RNA, ribozyme, DNAzyme and mixtures thereof, and is preferably mRNA, but is not limited to the above-mentioned examples.

The nucleic acid is for preventing or treating a disease, and for example, like a COVID-19 vaccine, enables synthesis of spike protein to fight COVID-19 viruses. However, the nucleic acid is not limited to the above-mentioned examples, and nucleic acids for preventing or treating a disease may all be used.

After that, an ionizable lipid is dissolved in an organic solution to prepare a first oil phase solution. As the ionizable lipid, ALC-0315 (Genevant), ALC-0159 (Genevant), DLinDAP, Dlin-MC3-DMA, SM102 (Arbutus) or the like may be used. The ionizable lipid is not limited to the above-mentioned examples, and ionizable lipids usable in manufacture of lipid nanoparticles may all be used without limit.

The organic solution is an alcohol, and may specifically be methanol, ethanol, isopropanol, n-propanol or the like. The organic solution is preferably ethanol, but is not limited to the above-mentioned examples, and organic solvents capable of uniformly dissolving an ionizable lipid may all be used without limit.

A non-ionizable lipid, a neutral lipid and a conjugated lipid are dissolved in an organic solution to prepare a second oil phase solution.

The non-ionizable lipid may be included together with the conjugated lipid in order to increase stability of lipid nanoparticles. Lipid nanoparticles are used to enable nucleic acid to reach a target tissue or organ, but have a problem of, after being injected into the body, being destroyed before reaching a target tissue or organ. In order to prevent such a problem, a non-ionizable lipid and a conjugated lipid may be included. Specifically, the non-ionizable lipid is selected from the group consisting of DSPC (distearoylphosphatidylcholine), DOPE (dioleolphosphatidyl ethanolamine), DPPE (bis(diphenylphosphino)ethane), diacyl phosphatidylcholine, diacylphosphatidylethanolamine, diacylphosphatidylserine and mixtures thereof, and is preferably DSPC, but is not limited to the above-mentioned examples.

The conjugated lipid may be selected from the group consisting of cholesterol, tocopherol and mixtures thereof, and is preferably cholesterol, but is not limited to the above-mentioned examples.

The neutral lipid is included to control particle sizes and to act as a steric barrier preventing aggregation during storage, and may specifically be selected from the group consisting of polyethylene glycol 2000 distearoylphosphatidylethanolamine (PEG(2000) DSPE), DMG-PEG, PEG-DMPE, DPPE-PEG, DPG-PEG, PEG-DOPE and mixtures thereof, and is preferably DMG-PEG, but is not limited to the above-mentioned examples.

The organic solution used for preparing the second oil phase solution is an alcohol and may specifically be methanol, ethanol, isopropanol, n-propanol or the like, and is preferably ethanol. However, the organic solution is not limited to the above-mentioned examples, and organic solvents capable of uniformly dissolving an ionizable lipid may all be used without limit.

The prepared aqueous phase solution and first oil phase solution are respectively injected into a first channel and a second channel to flow therethrough. The first channel and the second channel form an intersection as to be described later, and is connected to a stirring channel.

The aqueous phase solution and the first oil phase solution respectively injected into the first channel and the second channel form a laminar flow at the intersection and flow through the stirring channel.

In a general process for manufacturing lipid nanoparticles, an aqueous phase solution in which nucleic acid is dissolved and an oil phase solution in which four types of lipids are all dissolved are injected into each channel, and the aqueous phase solution and the oil phase solution injected into the channels form a laminar flow at the intersection and flow through a stirring channel. The aqueous phase solution and the oil phase solution injected into the stirring channel are mixed, and the nucleic acid in the aqueous phase solution, and the ionizable lipid, the non-ionizable lipid, the neutral lipid and the conjugated lipid in the oil phase solution bind by electrostatic attraction to form lipid nanoparticles.

In other words, lipid nanoparticles are manufactured by mixing the aqueous phase solution and the oil phase solution, and for the oil phase solution, a state in which all four types of lipids are mixed is used.

On the other hand, in the method for manufacturing lipid nanoparticles including a low concentration of ionizable lipid of the present disclosure, the oil phase solution is divided into a first oil phase solution and a second oil phase solution, and the first oil phase solution includes only an ionizable lipid and the second oil phase solution includes remaining lipids.

As described above, the aqueous phase solution flows in the first channel and the first oil phase solution flows in the second channel, and the nucleic acid in the aqueous phase solution and the ionizable lipid in the first oil phase solution first bind by electrostatic attraction.

In order for the nucleic acid in the aqueous phase solution and the ionizable lipid in the first oil phase solution to more readily bind by electrostatic attraction, the solutions pass through a stirring unit in the stirring channel.

Specifically, the stirring channel includes a plurality of mixing modules including the stirring unit and a non-stirring unit.

In the mixing module, a $n^{th}$ mixing module is formed in order based on a fluid flow direction of the stirring channel, and the $n^{th}$ mixing module means an order of the mixing module repeatedly formed in the stirring channel.

Specifically, the first mixing module is formed in the very front based on a fluid flow direction of the stirring channel, and after that, the second mixing module, the third mixing module and the like may be formed.

The mixing module includes a stirring unit and a non-stirring unit. As to be described later, a groove is formed in the stirring unit in order for the aqueous phase solution and the first oil phase solution to be well mixed. While the aqueous phase solution and the first oil phase solution flow after forming a laminar flow, they are chaotic mixed in the stirring unit, and the nucleic acid and the ionizable lipid bind by the mixing process.

The nucleic acid is specifically mRNA, and mRNA is anionic as described above, and the ionizable lipid is cationic, and they bind by mutual electrostatic attraction.

A mixing process is performed while the aqueous phase solution and the first oil phase solution pass through the stirring channel, and when they flow through the stirring channel as a first mixture solution, a second mixture solution is sequentially injected into a third channel to be mixed with the first mixture solution in the stirring channel.

The third channel forms an intersection with the stirring channel between the first mixing module to the fifth mixing module and is coupled therewith. Specifically, the third channel forms an intersection with the stirring channel between the second mixing module to the fourth mixing module and is coupled therewith, and more preferably, forms an intersection with the stirring channel in a non-stirring unit in the third mixing module and is coupled therewith.

As described above, the coupled portion of the third channel is determined considering the degree of binding between the nucleic acid and the ionizable lipid so that, before the second oil phase solution is mixed with the first mixture solution by forming a laminar flow, the nucleic acid and the ionizable lipid completely bind in the first mixture solution and then bind to the non-ionizable lipid, the neutral lipid, and the conjugated lipid in the second oil phase solution.

Specifically, it is identified that, when forming a laminar flow by crossing the aqueous phase solution and the first oil phase solution using the first channel and the second channel, then flowing through the stirring channel and then passing through the mixing module, the nucleic acid in the aqueous phase solution and the ionizable lipid in the first oil phase solution are mixed after passing through a stirring unit in the third mixing module and a mixing rate of 80% or greater is obtained.

Specifically, the aqueous phase solution injected into the first channel and the first oil phase solution injected into the second channel may have a flow rate ratio of 1:1 to 10:1 and 3:1 to 9:1. When mixed and used in the above-mentioned range, lipid nanoparticles with a uniform particle size may be manufactured. More specifically, the flow rate ratio being less than the above-mentioned range has a problem in that it is difficult to maintain a lipid nanoparticle shape during a subsequent process by having a high ethanol content, and the flow rate ratio being greater than the above-mentioned range has a problem of forming particles having a too small diameter since the movement of lipid particles in the aqueous phase solution is limited by including a large amount of the aqueous phase solution.

In view of the above, the present disclosure determines a coupled position of the third channel with the stirring channel considering the degree of binding between the nucleic acid and the ionizable lipid, and the second oil phase solution forms a laminar flow with the first mixture solution by coupling the third channel with a non-stirring unit in the first mixing module, a non-stirring unit in the second mixing module, a non-stirring unit in the third mixing module, a non-stirring unit in the fourth mixing module or a non-stirring unit in the fifth mixing module, and after that, the second oil phase solution and the first mixture solution are mixed by passing through the stirring module to form a second mixture solution, and in the second mixture solution, particles in which the nucleic acid and the ionizable lipid bind, and the non-ionizable lipid, the neutral lipid and the conjugated lipid bind to form lipid nanoparticles including nucleic acid.

Compared to when mixing nucleic acid, an ionizable lipid, a non-ionizable lipid, a neutral lipid and a conjugated lipid at once to manufacture lipid nanoparticles, more uniform lipid nanoparticles may be manufactured when lipid nanoparticles are formed by inducing stepwise binding as in the present disclosure unlike the related art.

The lipid nanoparticles manufactured in the present disclosure have a uniform spherical shape, and may have a polydispersity index of 0.2 or less, 0.01 to 0.2, 0.05 to 0.2, and 0.1 to 0.2. Lipid nanoparticles satisfying the polydispersity index in the above-mentioned range means that lipid nanoparticles having a very uniform size are provided. When manufacturing lipid nanoparticles having a uniform size as above, a separate operation for sorting lipid nanoparticles having specific sizes is not required, and therefore, a production yield may be maximized in a subsequent process after the mixing process for providing the lipid nanoparticles as a vaccine or therapeutic.

In addition, the lipid nanoparticles including nucleic acid of the present disclosure may include the ionizable lipid in an amount of 10 mol% to 30 mol% with respect to the total lipid weight in the lipid nanoparticles. In addition, the ionizable lipid may be included in an amount of 15 mol% to 19.9 mol% with respect to the total lipid weight in the lipid nanoparticles. Even when including the ionizable lipid in an amount of 20 mol% or greater as above, lipid nanoparticles having a uniform diameter may be manufactured compared to existing methods for manufacturing lipid nanoparticles, and the ionizable lipid content range allows the manufactured lipid nanoparticles to have a uniform size even when included in a low concentration compared to mRNA vaccines commercialized as well as in the prior patents examined above. In other words, while resolving the problem of toxicity by lowering the content of ionizable lipid having toxicity issues, the ionizable lipid and other lipids are separated in the manufacturing process before binding to the nucleic acid, and as a result, lipid nanoparticles having a uniform size may be manufactured, which may increase the production yield.

The ionizable lipid and the nucleic acid may have a weight ratio of 3:1 to 50:1, 3.3:1 to 50:1 and 3.3:1 to 16.7:1. In addition, the ionizable lipid, the non-ionizable lipid, the conjugated lipid and the neutral lipid may have a molar ratio in the range of 10 to 50:10 to 50:30 to 65:1 to 2.5. Compared to the related art, it may be identified that the content range of the conjugated lipid increases, and the content of the ionizable lipid decreases.

As described above, manufactured lipid nanoparticles do not have a uniform size when lowering an ionizable lipid content in an existing manufacturing method, and it is known that it is virtually impossible to adjust the content ranges of lipids.

However, unlike the existing manufacturing method, lipids are classified into an ionizable lipid and remaining lipids in the present disclosure, and after dissolving each of these in each organic solvent, they bind with nucleic acid in a stepwise manner, and as a result, lipid nanoparticles having a uniform size may be manufactured while lowering a content of the ionizable lipid.

Lipid nanoparticles including a low concentration of ionizable lipid according to another embodiment of the present disclosure may be manufactured using the above-described manufacturing method.

As described above, lipid nanoparticles manufactured using the manufacturing method of the present disclosure include a low content of ionizable lipid, and accordingly, a problem of toxicity caused by including a high content of ionizable lipid may be resolved, and the production yield may be increased since manufactured particles have a uniform size as well.

An apparatus for manufacturing lipid nanoparticles including a low concentration of ionizable lipid according to another embodiment of the present disclosure may include: a first channel through which an aqueous phase solution including nucleic acid flows; a second channel through which a first oil phase solution including an ionizable lipid flows; a third channel through which a second oil phase solution including a non-ionizable lipid, a neutral lipid and a conjugated lipid flows; and a stirring channel, wherein the first channel and the second channel form a first intersection, the intersection is connected to the stirring channel, and the stirring channel may include a mixing module of a stirring unit and a non-stirring unit.

The mixing module is formed in plural in the stirring channel, and an $n^{th}$ mixing module is formed in order based on a fluid flow direction of the stirring channel, and the $n^{th}$ mixing module means an order of the mixing module repeatedly formed in the stirring channel.

The number of the mixing modules included in the stirring channel may be from 3 to 70. As described above, the mixing module includes a stirring unit and a non-stirring unit, so that the aqueous phase solution and the oil phase solution flowing as a laminar flow are mixed in the stirring unit, and by having a plurality of the stirring units and the non-stirring units to be repeated in the stirring channel, mixing efficiency may be increased.

The number of the mixing modules included in the stirring channel may be from 3 to 70, 3 to 50, 3 to 40, 3 to 35, and preferably 30. When the mixing module is included in the above-mentioned range, the nucleic acid in the aqueous phase solution and the ionizable lipid in the first oil phase solution completely bind, and then bind with the non-ionizable lipid, the neutral lipid and the conjugated lipid in the second oil phase solution in a stepwise manner as described above, and as a result, lipid nanoparticles having a uniform size may be manufactured.

The third channel may be coupled with the stirring channel by forming an intersection between the first mixing module to the fifth mixing module.

The third channel is for flowing the second oil phase solution to form a laminar flow with the first mixture solution flowing through the stirring channel, and as described above, the lipids in the second oil phase solution flowing through the third channel bind to the stirring channel at a predetermined distance with the intersection between the first channel and the second channel so as to bind to, after forming particles by sufficiently mixing the nucleic acid and the ionizable lipid, the particles.

The coupled position of the third channel is between the first mixing module to the fifth mixing module, and specifically, the third channel may be coupled with a non-stirring unit in the first mixing module, a non-stirring unit in the second mixing module, a non-stirring unit in the third mixing module, a non-stirring unit in the fourth mixing module or a non-stirring unit in the fifth mixing module, may be preferably coupled with a non-stirring unit in the second mixing module, a non-stirring unit in the third mixing module, a non-stirring unit in the fourth mixing module or a non-stirring unit in the fifth mixing module, and may be more preferably coupled with a non-stirring unit in the third mixing module.

By adjusting the coupled position as above, the first mixture solution is mixed through a stirring unit of the first mixing module, a stirring unit of the second mixing module and a stirring unit of the third mixing module before the second oil phase solution in the third channel intersects with the first mixture solution, and sufficient time may be provided for binding between the nucleic acid in the aqueous phase solution and the ionizable lipid in the first oil phase solution.

The mixing module may have a length of 1 mm to 5 mm based on a fluid flow direction in the stirring channel. In addition, the stirring unit and the non-stirring unit may have a length ratio of 45:1 to 5:0.3 based on a fluid flow direction in the stirring channel. Specifically, the stirring unit may have a length of 2 mm to 4 mm and the non-stirring unit may have a length of 0.1 mm to 0.25 mm, the stirring unit may have a length of 2 mm to 3.5 mm and the a non-stirring unit may have a length of 0.1 mm to 0.20 mm, and the stirring unit may have a length of 2 mm to 3 mm and the non-stirring unit may have a length of 0.12 mm to 0.20 mm. By the stirring unit and the non-stirring unit forming the mixing module in the above-mentioned range, the nucleic acid and the lipids readily bind in the first mixture solution and the second mixture solution passing through the stirring unit and the non-stirring unit.

A groove is formed in the stirring unit to mix the introduced fluids, and the groove of the stirring unit may generate chaotic mixing in order to increase mixing efficiency of a laminar flow in the stirring channel.

The groove may have a shape of rectangle, semicircle or triangle. The shape of the groove is for increasing mixing efficiency of the fluids passing through the stirring channel, and groove shapes capable of increasing stirring efficiency may all be used without being limited to the above-mentioned examples.

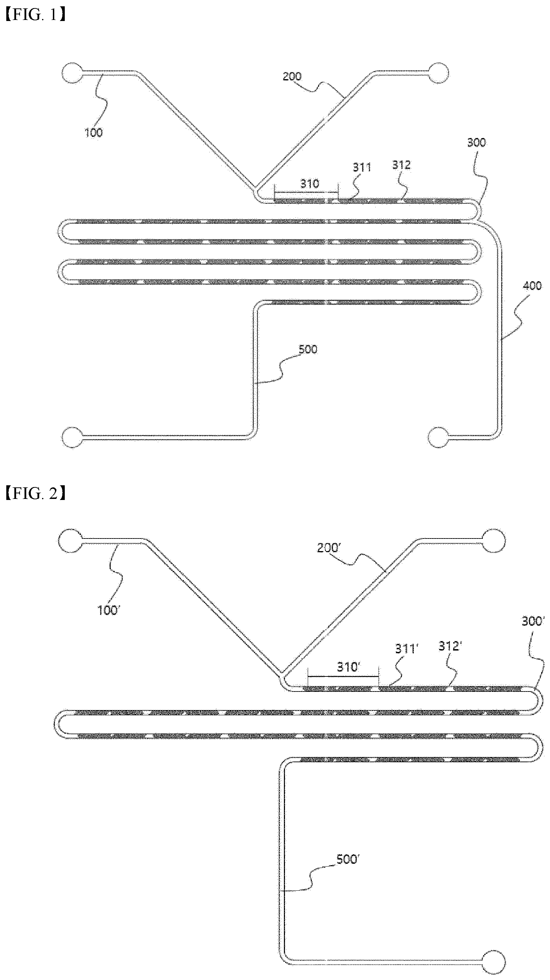

Specifically, FIG. 1 relates to an apparatus for manufacturing lipid nanoparticles of the present disclosure. Specifically, the apparatus for manufacturing lipid nanoparticles of the present disclosure includes a first channel 100, a second channel 200, a stirring channel 300 and a third channel 400, and a mixing module 310 is repeatedly formed in the stirring channel 300.

In addition, a stirring unit 311 and a non-stirring unit 312 are formed in the mixing module 310.

FIG. 2 relates to an existing apparatus for manufacturing lipid nanoparticles, and unlike FIG. 1, a third channel 400' is not separately included in a stirring channel 300'.

The manufacturing apparatus may be formed on a material selected from the group consisting of a glass substrate, a silicon wafer and a polymer film, however, examples of the material are not limited to the above-mentioned examples, and materials capable of forming microchannels may all be used.

The polymer film may be selected from the group consisting of polyimide, polyethylene, fluorinated ethylene propylene, polypropylene, polyethylene terephthalate, polyethylene naphthalate, polysulfone and mixtures thereof, but is not limited to the above-mentioned examples.

For example, aluminum is deposited on a silicon wafer using an e-beam evaporator, and a photoresist is patterned on the aluminum using a photolithography method. After that, the aluminum is etched using the photoresist as a mask, and after removing the photoresist, the silicon is etched by deep ion reactive etching using the aluminum as a mask, and after removing the aluminum, glass is anodically bonded on the wafer to seal, and as a result, the manufacturing apparatus is manufactured.

In the manufacturing apparatus, the first channel, the second channel, the third channel and the stirring channel have an average diameter of 180 $\mu$m to 220 $\mu$m and preferably 200 $\mu$m, have a height of 60 $\mu$m to 100 $\mu$m and preferably 80 $\mu$m, and have a groove height of 10 $\mu$m to 50 $\mu$m and preferably 30 $\mu$m, however, channel diameters, heights and groove heights capable of manufacturing lipid nanoparticles by solution flow may all be used without limit.

The aqueous phase solution, the first oil phase solution and the second oil phase solution injected into the channels are injected at flow rates of 0.3 ml/min to 0.9 ml/min, 0.05 ml/min to 0.3 ml/min and 0.05 ml/min to 0.3 ml/min, respectively. When the aqueous phase solution, the first oil phase solution and the second oil phase solution are injected in the above-mentioned range, lipid nanoparticles having a uniform size may be manufactured.

In addition, as described above, the nucleic acid and the ionizable lipid need to be sufficiently bind in the first mixture solution before the second oil phase solution intersects to be mixed with the first mixture solution. In order to increase the binding rate (mixing index rate) between the nucleic acid and the ionizable lipid, the flow rates of the aqueous phase solution and the first oil phase solution injected into the channels need to be controlled, and herein, the flow rate ratio may be from 1:1 to 10:1, preferably from 2:1 to 10:1 and more preferably from 3:1 to 9:1. Within the above-mentioned flow rate ratio range, the mixing index rate of the nucleic acid and the ionizable lipid in the first mixture solution passing through the stirring unit in the third mixing module may be 80% or greater and 85% or greater. Satisfying the mixing index rate value as above means that the nucleic acid and the ionizable lipid sufficiently bind, and after that, the mixed nucleic acid and ionizable lipid may bind to other lipids through the third channel, and manufactured into lipid nanoparticles.

Preparation Example 1

Manufacture of Apparatus for Manufacturing Lipid Nanoparticles

A negative photoresist was coated on a surface of a silicon wafer through rotation, then the result was heated for 0 to 3 minutes at 65°C and 6 to 9 minutes at 95°C to evaporate the solvent, and then ultraviolet rays was irradiated thereon except the groove structure. After that, the ultraviolet-exposed portion of the photoresist was solidified through heating for 1 to 2 minutes at 65°C and 6 to 7 minutes at 95°C.

In order to form a groove in a stirring channel, a negative photoresist was coated through rotation once more, then the result was heated for 0 to 3 minutes at 65°C and 6 to 9 minutes at 95°C to evaporate the solvent, and then ultraviolet rays were irradiated on a portion for the groove of the chip. After that, the ultraviolet-exposed portion of the photoresist was solidified through heating for 1 to 2 minutes at 65°C and 6 to 7 minutes at 95°C. After that, the portion not exposed to ultraviolet rays was removed using a developer.

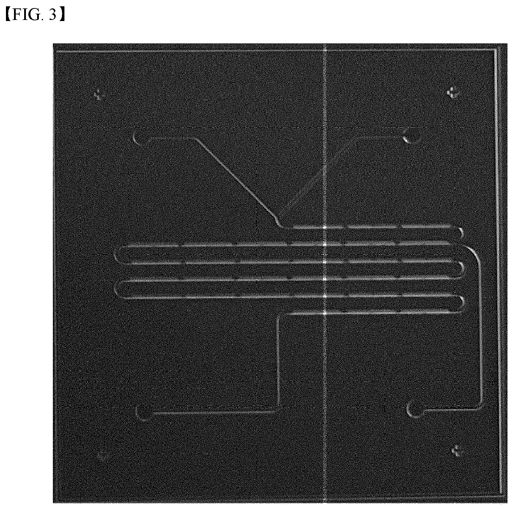

The manufactured manufacturing apparatus is shown in FIG. 3.

Preparation Example 2

Manufacture of Lipid Nanoparticles

mRNA (CleanCap® Firefly Luciferase mRNA, ~1,929 nucleotides) was mixed with a 10 mM citrate solution (pH 3) to prepare an aqueous phase solution.

As an ionizable lipid, ALC-0315 was dissolved in ethanol to prepare a first oil phase solution. After that, DSPC, cholesterol and DMG-PEG2000 were dissolved in ethanol to prepare a second oil phase solution.

The aqueous phase solution was injected into the first channel of the manufacturing apparatus manufactured in Preparation Example 1, the first oil phase solution was injected into the second channel, and the second oil phase solution was injected into the third channel.

The aqueous phase solution was injected at a flow rate of 0.6 mL/min, the first oil phase solution at 0.1 mL/min, and the second oil phase solution at 0.1 mL/min.

As for the solution discharged out of the apparatus after completing the mixing, the buffer solution was exchanged with PBS through dialysis (PES membrane dialysis cassette (MWCO=10,000 dalton)), and as a result, lipid nanoparticles were manufactured.

The contents of the constituents for the lipid nanoparticles are shown in the following Table 1.

[Table 1]

| | Mixing Ratio between Lipids (mol%) | | | | ALC-0315:mRNA (Weight%) |
|---|---|---|---|---|---|
| | ALC-0315 | DSPC | Cholesterol | DMG-PEG2000 | |
| Example 1 | 16.5 | 16.7 | 64.3 | 2.5 | 3.3:1 |
| Comparative Example 1 | 16.5 | 16.7 | 64.3 | 2.5 | 3.3:1 |
| Example 2 | 37.4 | 12.5 | 48.2 | 1.9 | 10:1 |
| Example 3 | 30 | 30 | 38.5 | 1.5 | 16.7:1 |
| Comparative Example 2 | 30 | 30 | 38.5 | 1.5 | 16.7:1 |

In Comparative Examples, a manufacturing apparatus with no third channel as in FIG. 2 was used as the manufacturing apparatus, and preparation was made in the same manner as in Preparation Example except that ALC-0315, DSPC, cholesterol and DMG-PEG2000 were dissolved in ethanol and injected into the second channel as the oil phase solution.

As for Example 3 and Comparative Example 2, preparation was made in the same manner as in Preparation Example 2 except that, as the mRNA, CleanCap® Enhanced Green Fluorescent Protein mRNA (996 nucleotides) was used.

Experimental Example 1

Evaluation on Particle Size Distribution of Particles

For the lipid nanoparticles manufactured in each of Preparation Example 2 and Comparative Example 1, particle size distribution (polydispersity index) of the particles was measured using a dynamic laser scattering device (Malvern Zetasizer).

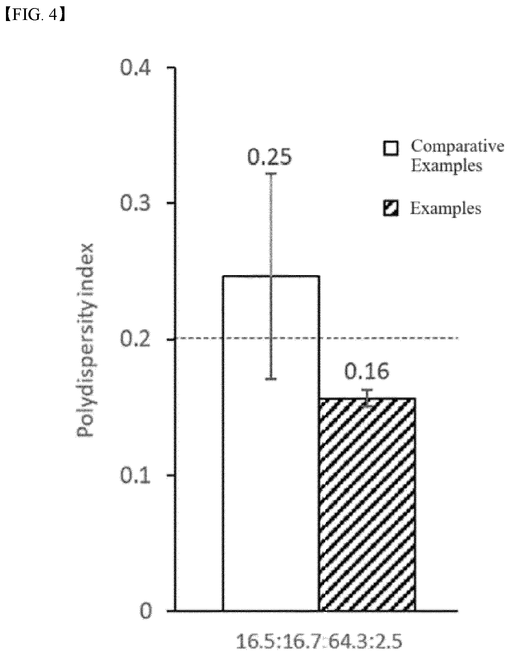

The experimental results are shown in FIG. 4.

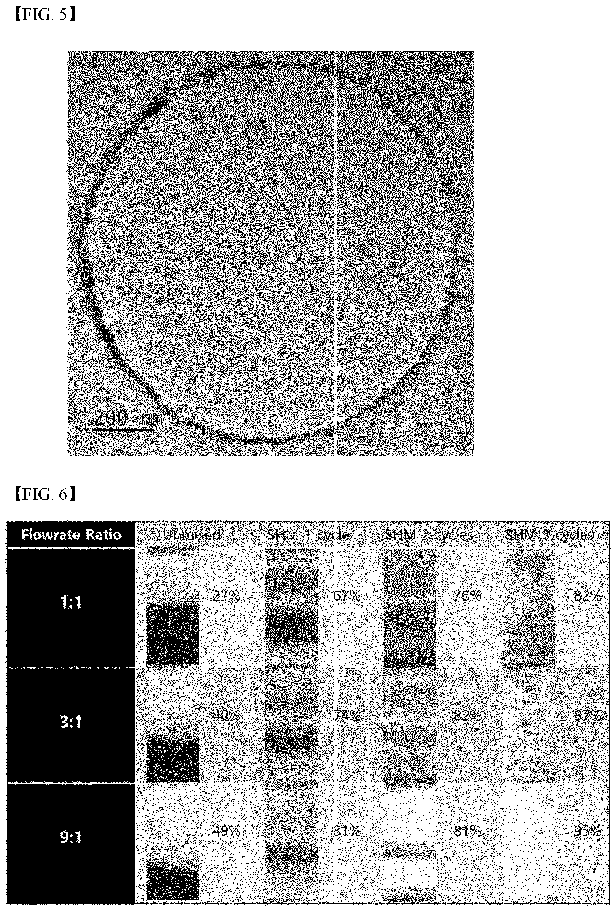

Specifically, when including 16.5 mol% of the ionizable lipid, the lipid nanoparticles manufactured using the manufacturing apparatus of Comparative Examples had a PDI of 0.25±0.08, whereas the lipid nanoparticles manufactured using the manufacturing apparatus of Preparation Examples had a PDI of 0.15±0.01, and it was identified that, as in FIG. 5, uniform-sized lipid nanoparticles were manufactured.

In addition, Example 2 was prepared using the manufacturing apparatus of the present disclosure while increasing the content of the ionizable lipid, and the PDI value was identified to be 0.08±0.03 which is 0.2 or less.

Example 3 and Comparative Example 2 were prepared in the same manner using different mRNAs, and Example 3 was identified to have a PDI of 0.15 and Comparative Example 2 was identified to have a PDI of 0.24, and it was identified that there was a difference in the diameter uniformity in the prepared particles.

Experimental Example 2

SHM Mixing Evaluation

In order to identify the degree of stirring of the aqueous phase solution and the first oil phase solution, an alternative experiment was conducted. Through the experiment, binding between the mRNA in the aqueous phase solution and the ionizable lipid in the first oil phase solution was identified.

Specifically, instead of the aqueous phase solution, DI Water was mixed with Rhodamine B in 0.015% w/w to prepare an aqueous phase solution, and, instead of the first oil phase solution, ethanol was mixed with Rhodamine B in 0.015% w/w to prepare an oil phase solution.

The aqueous phase solution and the oil phase solution were injected into the first channel and the second channel, respectively. The flow rate and the flow rate ratio when injected into each of the channels are as follows.

[Table 2]

| TFR (µL/min) | Solution | Flow Rate (µL/min) | | |
|---|---|---|---|---|
| 300 | DI Water | 150 | 225 | 270 |
| | Ethanol | 150 | 75 | 30 |

(continued)

| TFR (µL/min) | Solution | Flow Rate (µL/min) | | |
|---|---|---|---|---|
| | FRR | 1:1 | 3:1 | 9:1 |

After the mixture solution was injected into the first channel and the second channel, a mixing index rate (%) obtained from the difference in the flow rate ratio was measured.

The following index image analysis method was used.

① Convert the photographed image file into Grey Scale using Image J
② Change the image converted to Grey Scale to 8 Bit
③ Background Intensity filtering operation
④ Analyze intensity and calculate index for each pixel using the following formula

$$MI = 1 - \sqrt{\frac{1}{N}\sum_{i=1}^{N}(\frac{c_i - \bar{c}}{\acute{c}})^2}$$

herein,

N is the total number of pixels,
$C_i$ is a pixel value of i,
$\bar{C}$ is an average value of complete pixels, and
$\acute{C}$ is an average value of unmixed pixels.

The experimental results are shown in FIG. 6.

When mixing the aqueous phase solution and the oil phase solution in a flow rate ratio of 1:1, the mixture solution passed through the mixing module 3 times and had a mixing index rate of 82%, however, when mixed in a ratio of 1:1, the degree of mixing was relatively low. This means that when using the aqueous phase solution and the oil phase solution to manufacture actual lipid nanoparticles, the mRNA in the aqueous phase solution and the ionizable lipid in the first oil phase solution may not sufficiently bind.

Accordingly, in the experiments, it was identified that, when the aqueous phase solution and the oil phase solution were mixed and prepared in a flow rate ratio of 3:1 to 9:1, the mixing degree of the solution was high of 87% and 95%, respectively, after passing through the third mixing module. Through the experiments, it can be identified that a preferred flow rate ratio for mixing the aqueous phase solution and the first oil phase solution is from 3:1 to 9:1.

| | | | |
|---|---|---|---|
| 100: | First Channel | 100': | First Channel |
| 200: | Second Channel | 200': | Second Channel |
| 300: | Stirring Channel | 300': | Stirring Channel |
| 310: | Mixing Module | 310': | Mixing Module |
| 311: | Stirring Unit | 311': | Stirring Unit |
| 312: | Non-Stirring Unit | 312': | Non-Stirring Unit |
| 400: | Third Channel | | |
| 500: | Outflow Channel | 500': | Outflow Channel. |

**Claims**

1. A method for manufacturing lipid nanoparticles, the method comprising:

preparing an aqueous phase solution including nucleic acid;

preparing a first oil phase solution by dissolving an ionizable lipid in an organic solution;

preparing a second oil phase solution by dissolving a non-ionizable lipid, a neutral lipid and a conjugated lipid in an organic solution;

injecting and flowing the aqueous phase solution into a first channel;

injecting and flowing the first oil phase solution into a second channel;

the aqueous phase solution and the first oil phase solution intersecting with each other to flow through a stirring channel as a first mixture solution;

flowing the second oil phase solution to a third channel connected to the stirring channel to intersect with the first mixture solution and be mixed as a second mixture solution; and

forming lipid nanoparticles including nucleic acid by passing the second mixture solution through a stirring unit in the stirring channel.

2. The method of claim 1, wherein the stirring channel includes a mixing module of the stirring unit and a non-stirring unit.

3. The method of claim 2, wherein the mixing module is formed in plural in the stirring channel, and a $n^{th}$ mixing module is formed in order based on a fluid flow direction of the stirring channel, and the $n^{th}$ mixing module means an order of the mixing module repeatedly formed in the stirring channel.

4. The method of claim 3, wherein the third channel forms an intersection between the first mixing module to the fifth mixing module to be coupled with the stirring channel, and the first mixture solution and the second oil phase solution intersect to form a laminar flow.

5. The method of any one of claims 1 to 4, wherein the ionizable lipid is included in an amount of 10 mol% to 30 mol% with respect to a total lipid weight in the lipid nanoparticles.

6. The method of any one of claims 1 to 5, wherein the ionizable lipid and the nucleic acid have a weight ratio of 3:1 to 50:1.

7. Lipid nanoparticles manufactured using the method of any one of claims 1 to 6, wherein the lipid nanoparticles have a uniform spherical shape, and have a polydispersity index of 0.2 or less.

8. An apparatus for manufacturing lipid nanoparticles, the apparatus comprising:

a first channel through which an aqueous phase solution including nucleic acid flows;

a second channel through which a first oil phase solution including an ionizable lipid flows;

a third channel through which a second oil phase solution including a non-ionizable lipid, a neutral lipid and a conjugated lipid flows; and

a stirring channel,

wherein the first channel and the second channel form a first intersection, and the intersection is connected to the stirring channel; and

the stirring channel includes a mixing module of a stirring unit and a non-stirring unit.

9. The apparatus of claim 8, wherein the mixing module is formed in plural in the stirring channel, and a $n^{th}$ mixing module is formed in order based on a fluid flow direction of the stirring channel, and the $n^{th}$ mixing module means an order of the mixing module repeatedly formed in the stirring channel.

10. The apparatus of claim 9, wherein the third channel forms an intersection between the first mixing module to the fifth mixing module to be coupled with the stirring channel.

11. The apparatus of any one of claims 8 to 10, wherein the mixing module has a length of 1 mm to 5 mm based on a fluid flow direction in the stirring channel.

12. The apparatus of any one of claims 8 to 10, wherein the stirring unit and the non-stirring unit have a length ratio of 45:1 to 5:0.3 based on a fluid flow direction in the stirring channel.

13. The apparatus of any one of claims 8 to 10, wherein a groove is formed in the stirring unit in order to mix the introduced fluids.

**14.** The apparatus of claim 13, wherein the groove of the stirring unit generates chaotic mixing in order to increase mixing efficiency of a laminar flow in the stirring channel.

## Patentansprüche

**1.** Ein Verfahren zur Herstellung von Lipidnanopartikeln, das Verfahren umfassend

Herstellen einer Lösung einer wässrigen Phase, die Nukleinsäure enthält;
Herstellen einer ersten Lösung einer Ölphase durch Lösen eines ionisierbaren Lipids in einer organischen Lösung;
Herstellen einer zweiten Lösung einer Ölphase durch Lösen eines nicht-ionisierbaren Lipids, eines neutralen Lipids und eines konjugierten Lipids in einer organischen Lösung;
Einleiten und Strömen der Lösung der wässrigen Phase in einen ersten Kanal;
Einleiten und Strömen der ersten Lösung der Ölphase in einen zweiten Kanal;
wobei die Lösung der wässrigen Phase und die erste Lösung der Ölphase sich miteinander kreuzen, um als eine erste Mischlösung durch einen Rührkanal zu strömen;
Strömen der zweiten Lösung der Ölphase zu einem dritten Kanal, der mit dem Rührkanal verbunden ist, um sich mit der ersten Mischlösung zu kreuzen und als eine zweite Mischlösung vermischt zu werden; und
Bildung von Lipidnanopartikeln, die Nukleinsäure enthalten, durch Durchleiten der zweiten Mischlösung durch eine Rühreinheit im Rührkanal.

**2.** Das Verfahren aus Anspruch 1, wobei der Rührkanal ein Mischmodul der Rühreinheit und eine Nicht-Rühreinheit umfasst.

**3.** Das Verfahren aus Anspruch 2, wobei das Mischmodul mehrfach im Rührkanal ausgebildet ist, und ein n-tes Mischmodul in Reihenfolge basierend auf einer Strömungsrichtung der Flüssigkeit im Rührkanal ausgebildet ist, wobei das n-te Mischmodul eine Reihenfolge des im Rührkanal wiederholt ausgebildeten Mischmoduls bezeichnet.

**4.** Das Verfahren aus Anspruch 3, wobei der dritte Kanal eine Kreuzung zwischen dem ersten bis zum fünften Mischmodul bildet, um mit dem Rührkanal gekoppelt zu sein, und wobei die erste Mischlösung und die zweite Lösung der Ölphase sich kreuzen, um eine laminare Strömung zu bilden.

**5.** Das Verfahren aus irgendeinem der Ansprüche 1 bis 4, wobei das ionisierbare Lipid in einer Menge von 10 Mol-% bis 30 Mol-%, bezogen auf das Gesamtgewicht der Lipide, in den Lipidnanopartikeln enthalten ist.

**6.** Das Verfahren aus irgendeinem der Ansprüche 1 bis 5, wobei das ionisierbare Lipid und die Nukleinsäure ein Gewichtsverhältnis von 3:1 bis 50:1 aufweisen.

**7.** Lipidnanopartikel, hergestellt mit Hilfe des Verfahrens aus irgendeinem der Ansprüche 1 bis 6, wobei die Lipidnanopartikel eine gleichmäßige kugelförmige Form aufweisen und einen Polydispersitätsindex von 0,2 oder weniger haben.

**8.** Eine Vorrichtung zur Herstellung von Lipidnanopartikeln, die Vorrichtung umfassend

einen ersten Kanal, durch den eine Lösung einer wässrigen Phase, die Nukleinsäure enthält, strömt;
einen zweiten Kanal, durch den eine erste Lösung einer Ölphase, die ein ionisierbares Lipid enthält, strömt;
einen dritten Kanal, durch den eine zweite Lösung einer Ölphase, die ein nichtionisierbares Lipid, ein neutrales Lipid und ein konjugiertes Lipid enthält, strömt; und
einen Rührkanal,
wobei der erste Kanal und der zweite Kanal eine erste Kreuzung bilden, und die Kreuzung mit dem Rührkanal verbunden ist; und
wobei der Rührkanal ein Mischmodul einer Rühreinheit und eine Nicht-Rühreinheit umfasst.

**9.** Die Vorrichtung nach Anspruch 8, wobei das Mischmodul mehrfach im Rührkanal ausgebildet ist, und ein n-tes Mischmodul in Reihenfolge basierend auf einer Strömungsrichtung der Flüssigkeit im Rührkanal ausgebildet ist, wobei das n-te Mischmodul eine Reihenfolge des im Rührkanal wiederholt ausgebildeten Mischmoduls bezeichnet.

**10.** Die Vorrichtung nach Anspruch 9, wobei der dritte Kanal eine Kreuzung zwischen dem ersten bis zum fünften Mischmodul bildet, um mit dem Rührkanal gekoppelt zu sein.

**11.** Die Vorrichtung nach irgendeinem der Ansprüche 8 bis 10, wobei das Mischmodul eine Länge von 1 mm bis 5 mm, basierend auf einer Strömungsrichtung im Rührkanal, aufweist.

**12.** Die Vorrichtung nach einem der Ansprüche 8 bis 10, wobei die Rühreinheit und die Nicht-Rühreinheit ein Längenverhältnis von 45:1 bis 5:0,3 aufweisen, basierend auf einer Strömungsrichtung in dem Rührkanal.

**13.** Die Vorrichtung nach einem der Ansprüche 8 bis 10, wobei in der Rühreinheit eine Nut ausgebildet ist, um die eingeführten Flüssigkeiten zu vermischen.

**14.** Die Vorrichtung nach Anspruch 13, wobei die Nut der Rühreinheit ein chaotisches Mischen erzeugt, um die Mischeffizienz einer laminaren Strömung im Rührkanal zu erhöhen.

**Revendications**

**1.** Procédé de fabrication de nanoparticules lipidiques, ledit procédé comprenant :

la préparation d'une solution en phase aqueuse contenant de l'acide nucléique ;
la préparation d'une première solution en phase huileuse par dissolution d'un lipide ionisable dans une solution organique ;
la préparation d'une deuxième solution en phase huileuse par dissolution d'un lipide non ionisable, d'un lipide neutre et d'un lipide conjugué dans une solution organique ;
l'injection et l'écoulement de la solution en phase aqueuse dans un premier canal ;
l'injection et l'écoulement de la première solution en phase huileuse dans un deuxième canal ;
la solution en phase aqueuse et la première solution en phase huileuse se croisant de manière à s'écouler dans un canal d'agitation comme première solution de mélange ;
l'écoulement de la deuxième solution en phase huileuse vers un troisième canal relié au canal d'agitation de manière à croiser la première solution de mélange et à être mélangée comme deuxième solution de mélange ; et
la formation de nanoparticules lipidiques contenant de l'acide nucléique par passage de la deuxième solution de mélange par une unité d'agitation dans le canal d'agitation.

**2.** Procédé selon la revendication 1, où le canal d'agitation comprend un module de mélange de l'unité d'agitation et une unité exempte d'agitation.

**3.** Procédé selon la revendication 2, où le module de mélange est formé en pluralité dans le canal d'agitation, et un n-ième module de mélange est formé suivant un ordre basé sur la direction d'écoulement de fluide du canal d'agitation, et le n-ième module de mélange exprime l'ordre du module de mélange formé de manière répétée dans le canal d'agitation.

**4.** Procédé selon la revendication 3, où le troisième canal forme une intersection entre le premier module de mélange et le cinquième module de mélange à relier au canal d'agitation, et la première solution de mélange et la deuxième solution en phase huileuse se croisent de manière à former un flux laminaire.

**5.** Procédé selon l'une des revendications 1 à 4, où le lipide ionisable est contenu dans une teneur de 10 %mol à 30 %mol par rapport au poids total du lipide dans les nanoparticules lipidiques.

**6.** Procédé selon l'une des revendications 1 à 5, où le lipide ionisable et l'acide nucléique ont un rapport de poids de 3:1 à 50:1.

**7.** Nanoparticules lipidiques fabriquées au moyen du procédé selon l'une des revendications 1 à 6, lesdites nanoparticules lipidiques ayant une forme sphérique homogène et un indice de polydispersité inférieur ou égal à 0,2.

**8.** Dispositif pour la fabrication de nanoparticules lipidiques, ledit dispositif comprenant :

un premier canal où s'écoule une solution en phase aqueuse contenant de l'acide nucléique ;

un deuxième canal où s'écoule une première solution en phase huileuse contenant un lipide ionisable ;
un troisième canal où s'écoule une deuxième solution en phase huileuse contenant un lipide non ionisable, un lipide neutre et un lipide conjugué ; et
un canal d'agitation,

où le premier canal et le deuxième canal forment une première intersection, et où ladite intersection est reliée au canal d'agitation ; et
où le canal d'agitation comprend un module de mélange d'une unité d'agitation et d'une unité exempte d'agitation.

9. Dispositif selon la revendication 8, où le module de mélange est formé en pluralité dans le canal d'agitation, et un n-ième module de mélange est formé suivant un ordre basé sur la direction d'écoulement de fluide du canal d'agitation, et le n-ième module de mélange exprime l'ordre du module de mélange formé de manière répétée dans le canal d'agitation.

10. Dispositif selon la revendication 9, où le troisième canal forme une intersection entre le premier module de mélange et le cinquième module de mélange de manière à être relié au canal d'agitation.

11. Dispositif selon l'une des revendications 8 à 10, où le module de mélange a une longueur de 1 mm à 5 mm sur la base de la direction d'écoulement du fluide dans le canal d'agitation.

12. Dispositif selon l'une des revendications 8 à 10, où l'unité d'agitation et l'unité exempte d'agitation ont un rapport de longueur de 45:1 à 5:0,3 sur la base de la direction d'écoulement du fluide dans le canal d'agitation.

13. Dispositif selon l'une des revendications 8 à 10, où une rainure est formée dans l'unité d'agitation afin de mélanger les fluides introduits.

14. Dispositif selon la revendication 13, où la rainure de l'unité d'agitation génère un mélange chaotique afin d'accroître l'efficacité de mélange d'un flux laminaire dans le canal d'agitation.

【FIG. 1】

【FIG. 2】

17

【FIG. 3】

【FIG. 4】

【FIG. 5】

【FIG. 6】

| Flowrate Ratio | Unmixed | | SHM 1 cycle | | SHM 2 cycles | | SHM 3 cycles | |
|---|---|---|---|---|---|---|---|---|
| 1:1 | | 27% | | 67% | | 76% | | 82% |
| 3:1 | | 40% | | 74% | | 82% | | 87% |
| 9:1 | | 49% | | 81% | | 81% | | 95% |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190093816 A1
- JP 2016028031 A
- KR 1020170126944 A
- WO 2018190423 A1
- US 9364435 B2
- EP 2279254 B1

**Non-patent literature cited in the description**

- **MD BUSCHMANN et al.** Nanomaterial Delivery Systems for mRNA Vaccines. *Vaccines*, 2021, vol. 9 (65), 1-30
- **N KIMURA et al.** Development of a Microfluidic-Based Post-Treatment Process for Size-Controlled Lipid Nanoparticles and Application to siRNA Delivery. *ACS Appl. Mater. Interfaces*, 2020, vol. 12 (30), 34011-34020